# EUROPEAN PATENT APPLICATION

(11) **EP 4 006 142 A1**
(43) Date of publication of application: **01.06.2022**
(21) Application number: 20209895.0
(22) Date of filing: 25.11.2020
(51) Int. Cl.: C12N 5/071, G02B 21/00, G01N 21/21, G01N 21/23, G01N 15/14

(54) **ANALYSIS OF SEMINAL SAMPLES, METHOD AND SYSTEM**

(71) Applicant: Pera Labs Medikal Arastirma ve Gelistirme Limited Sirketi, 34467 Istanbul (TR)
(72) Inventor: Ozkosem, Burak, 34467 Istanbul (TR)
(74) Representative: Stellbrink & Partner Patentanwälte mbB

(57) **Abstract**

The present invention relates to a system for processing a seminal sample, the system comprising: a detecting component configured to detect sperm cells in the seminal sample, an analyzing component configured to analyze sperm cells in the seminal sample, and a sorting component configured to sort sperm cells in the seminal sample, wherein the system is configured to detect health status of the sperm cells. The present invention also relates to a method for analyzing a seminal sample, the method comprising: segregating sperm cells of the seminal sample using a device, detecting the sperm cells in the seminal sample by means of a detecting component, analyzing the sperm cells in the seminal sample by means of an analyzing component, sorting the sperm cells in the seminal sample by means of a sorting component, and recovering the sperm cells from the device.

## Description

### Field

The invention lies in the field of biological sample analysis and particularly in the field of mammal fluid analysis. The goal of the invention is to provide a system and a method for analysis of mammal seminal samples. More particularly, the present invention relates to a system for processing seminal sample, a method performed in such a system and corresponding use of the system.

### Introduction

Infertility is an increasing medical and social problem from which 8%-12% of couples suffer worldwide. Male factor infertility is an important cause of infertility, and approximately 40%-50% of all infertility cases are due to male infertility. Furthermore, the fertility rate among men younger than 30 years has decreased by 15% worldwide. A proper diagnosis of male factor infertility in an infertility analysis is dependent on an accurate measurement of semen quality. Semen quality analysis is the most frequently used diagnostic option and fundamental investigation in an infertility analysis. In addition to evaluating male infertility, semen analysis may also be used for post-vasectomy screening, and anyone who plans to preserve or donate sperm is expected to undergo such an examination. However, the shortcomings of conventional manual semen analysis (MSA), including that it is laboratory-based, labor intensive and subjective in nature, producing inconsistent reports that can cause misdiagnosis or delayed infertility treatment, have promoted the development of semi-computerized and computerized semen measuring devices, known as computer-assisted semen analysis (CASA) The conventional CASA systems are semi-automated sperm tracking computer-aided devices, essentially focusing through the microscope to provide successive images of spermatozoa within static field of view. These systems use special software to extract desired information and produce the desired output. If appropriate protocols are followed, high precision and provision of quantitative data on sperm kinetics are two major advantages of CASA over MSA. In addition, CASA systems reduce the burden of measuring sperm tracks when individual tracking data are needed. Another advantage is that any test samples can be analyzed in a short time. Most CASA systems allow partial automation in routine sperm analysis, but they have also shown limited success due to poor accuracy at low and high sperm counts and the high cost of virtually all CASA devices. Moreover, different CASA systems use different mathematical algorithms to calculate various sperm quality parameters, leading to the greatest disadvantage in the clinical application of CASA, with unreliability of comparative parameters across all device. Although CASA provides quantitative data on sperm kinetics, most CASA systems still rely greatly on highly trained technicians and require an additional bulky component for data collection and computational units for data analysis. The factors influencing the accuracy of the results and the lack of quality control may lead to large variations between different CASA systems and laboratories

WO 2016 182 551 A1 discloses a method and system for anatomical landmark detection in medical images using deep neural networks is disclosed. For each of a plurality of image patches centered at a respective one of a plurality of voxels in the medical image, a subset of voxels within the image patch is input to a trained deep neural network based on a predetermined sampling pattern. A location of a target landmark in the medical image is detected using the trained deep neural network based on the subset of voxels input to the trained deep neural network from each of the plurality of image patches.

US 2016 0174902 A1 discloses a method and system for anatomical object detection using marginal space deep neural networks is disclosed. The pose parameter space for an anatomical object is divided into a series of marginal search spaces with increasing dimensionality. A respective sparse deep neural network is trained for each of the marginal search spaces, resulting in a series of trained sparse deep neural networks. Each of the trained sparse deep neural networks is trained by injecting sparsity into a deep neural network by removing filter weights of the deep neural network.

US 8 639 043 B2 discloses methods for classifying different parts of a sample into respective classes based on an image stack that may comprise one or more images.

US2017 0053 398 A1 discloses various arrangements for identifying and grading cancer in tissue samples. A digital image of a stained tissue sample may be acquired. A Shearlet transform may be performed on the digital image of the stained tissue sample. Shearlet coefficients may be calculated based on the performed Shearlet transform of the normalized digital RGB image of the stained tissue sample. A trained neural network may be applied to create a plurality of feature maps using the digital image and Shearlet coefficients, wherein the trained neural network was trained using a plurality of images and Shearlet coefficients of a plurality of digital images. A classifier may be applied to an output of the trained neural network to identify whether cancer is present in the stained tissue sample. A notification may be output that is indicative of a grade of detected cancer in the sample.

### Summary

In light of the above, it is therefore an object of the present invention to overcome or at least to alleviated the shortcomings and disadvantages of the prior art. More particularly, it is an object of the present invention to provide a method and a corresponding system for analyzing seminal samples of mammals more effectively and less prompt to errors.

These objects are met by the present invention.

In a first aspect, the invention relates to a system for processing a seminal sample, the system comprising: a detecting component configured to detect sperm cells in the seminal sample, an analyzing component configured to analyze sperm cells in the seminal sample, and a sorting component configured to sort sperm cells in the seminal sample.

The system may be configured to detect health status of the sperm cells. Moreover, the system may further comprise at least one optical device. Furthermore, the system may comprise at least one linear polarizer.

In one embodiment, the system may comprise at least two variable retarders. Moreover, the system may comprise at least one light source.

The system may be configured to receive a disposable cartridge. The disposable cartridge may be a detachable and replaceable cartridge. The system may comprise a base component. The system may further comprise a microfluidic device configured to receive at least one portion of the seminal sample.

The cartridge may comprise a sample carrier, wherein the sample carrier may comprise a specimen holding area. The specimen holding area may comprise a top side and a bottom side, wherein the top side may be opposite to the bottom side. In another embodiment, the specimen holding area may comprise a detachable cover configured to cover the specimen holding area, wherein detachable cover may be arranged on the top side of the specimen holding area.

The disposable cartridge also may comprise a microfluidic element fluidly connected with the disposable cartridge. The disposable cartridge may comprise at least one identification mechanism. The at least one identification mechanism may comprise a barcode.

The system may comprise a fluid connection between the microfluidic device and the chamber. In one embodiment, the fluid connection may be a permanent fluid connection. In another embodiment, the fluid connection may be a direct fluid connection.

The at least one valve may be configured to selectively assume at least one of at least two operational states. The at least two operational states may comprise a first operational state and a second, wherein the system may be configured to establish a fluid connection between the microfluidic element and the disposable cartridge in the first operational state, and to discontinue the fluidic connection between the microfluidic element and the disposable cartridge while in the second operational state.

The microfluidic device may comprise a detection zone configured to receive the at least one portion of the seminal sample.

The system may be configured to analyzed the at least one portion of the seminal sample while the at least one portion of the seminal sample may be contained in the detection zone.

The base component may comprise an insertion port configured to allow inserting the disposable microfluidic device into the base component.

The base component may comprise at least one a camera. Moreover, the at least one optical device may comprise at least one lens system.

The at least one optical device may comprise a proximal section and a distal section.

The at least one lens system may be arranged in a portion of the proximal section of the at least one optical device. The at least one lens system may comprise a magnifying lens. The magnifying lens may be a microscope lens.

The at least one lens system may further comprise a central magnifying lens and an opaque material, wherein the opaque material surrounds the central magnifying lens.

The at least one optical device may comprise at least one sensor component arranged on specimen holding at a side of the at least one lens system.

The at least one sensor component may be configured to measure at least one signal passing through the seminal sample. The at least one signal may comprise a light signal, wherein the light signal passes from the seminal sample through the at least one lens system.

The sensor may comprise at least one of a complementary metal oxide semiconductor (CMOS), a charge-coupled device, a colorimeter, a contact image sensor, an electro-optical sensor, an infrared sensor, a fiber optic sensor, an optical position sensor, a photodetector, a photodiode, a photomultiplier tube, a phototransistor, a photoelectric sensor, a photoionization detector, a photoresistor, a photo-switch, and a photo-tube.

The at least one camera may be configured to capture at least one image data of the specimen holding area. The at least one image data may comprise a single frame image data. The at least one image data may comprise a sequence of consecutive individual frames comprising an image capture of at least 10 s⁻¹, preferably at least 90 s⁻¹, more preferably at least 300 s⁻¹, such as at least 600 s⁻¹.

The system may comprise at least two camera modules, wherein the system may comprise a first camera module configured to capture at least one first image data, and a second camera module configured to capture at least one second image. The first camera module may be arranged at a first position and the second camera module may be arranged at a second position, wherein the first position may be different from the second position. Moreover, the first camera module may be configured to capture the at least first image data at the first position and the second camera module may be configured to capture the at least second image data at the second position, wherein the first position may comprise at least a portion of the first holding area and the second position may comprise at least a portion of the second holding area.

The system may comprise a main circuit board comprising at least one processor unit configured to perform at least one analytical processing on the at least one image data to generate at least one analytical finding. The at least one processor unit may be configured to perform a first analytical processing on the at least first image data to generate at least one first analytical finding, and a second analytical processing on the at least second image data generate at least one second analytical finding. In one embodiment, first analytical processing may be different from the second analytical processing. In another embodiment, the first analytical processing and the second analytical processing may comprise an identical analytical processing. The at least one processor unit may be configured to determine a finding with regard to the biological specimen, wherein the finding may be based on at least one the at least one analytical finding. In one embodiment, the finding may be based on the at least first analytical finding. In another embodiment, the finding may be based on the at least second analytical finding. Additionally or alternatively, the finding may be based on the at least first analytical finding and the at least second analytical finding.

The system may comprise a housing configured to enclose at least one of: the at least one processor unit, the main circuit board, and at least one of the at least two cameras. In one embodiment, at least one of the at least two cameras may comprise a polarizer configured to facilitate birefringence measurement of the specimen.

The system may comprise at least one lighting system, wherein at least one of the least one lighting system may comprise at least one light source.

The lighting system may comprise optical fibers configured to transmit light from the at least one light source to the specimen holding area.

The system may comprise at least one transmitter configured to transmit data from the at least one processor unit to at least one remote device.

In one embodiment, at least one of the least one remote device may comprise at least one server.
The at least one transmitter may be configured to transmit data comprising information of the seminal sample about at least one of: sperm count, sperm morphology, sperm motility, and sperm DNA integrity.

The system may comprise at least one pump configured to transport at least a portion of the seminal sample from the macrofluidic fluidic chamber (disposable cartridge) into the microfluidic device.

In one embodiment, at least one of the least one pump may be an active pump. In another embodiment, at least one of the least one pump may be a passive pump. Furthermore, at least one of the at least one pump may be fluidly connected to the specimen holding area.

The system may be further configured to select sperm cells based on the sperm cells health status.

The system may be also configured to determine sperm concentration. The system may be further configured to determine sperm motility. The system may be further configured to determine sperm DNA integrity.

The system may comprise a built-in high-resolution and autofocus optical lens. The autofocus optical lens may be configured to capture image data with a capture-rate of at least 60 frames per second, at least 100 frames per second, at least 660 frames per second, at least 200 frames per second

The system may further comprise an artificial intelligence-based module configured to execute auto-calculations.

The system may be configured to execute a morphological analysis. The morphological analysis may be based on the captured image data.

In one embodiment, the at least one processor unit comprised by the analytical device may also be configured to receive optical data from the optical device. The at least one processor unit may have sufficient power to perform a high-level, single-thread object detection and/or tracking.

Moreover, the at least one processor unit may be single-core unit. In another embodiment, the at least one processor unit may be a multi-core unit. Furthermore, the processor may comprise a speed of less than about 5 GHz, less than 3 GHz, less than 3 GHz, less than 500 MHz, such as, for example, or less than 250 MHz.

The analytical device may comprise a power source. In one embodiment, the power source may be a battery.

Moreover, the analytical device may comprise a lighting system comprising a light source suitable for microscopy, such as a tungsten lamp, halogen lamp, mercury lamp, xenon lamp, or LED lamp.

In one embodiment, the lighting system may comprise optical fibers configured to transmit light from the light source to the receptacle. The optical fibers may focus the light on the microfilament element or on the detection zone of the microfilament. In another embodiment, the lighting system may be configured to illuminate the microfluidic chip, wherein the microfluid chip may comprise at one layer of a reflective coating on the walls of the microfluidic chip, wherein the at least one layer may be configured to focus the light on the detection zone of the microfluidic element.

The analytical device may comprise a transmitter configured to transmit data comprising information about at least one of sperm number, sperm morphology and sperm motility of the seminal sample to a remote device. For example, the analytical device may comprise a radio frequency transmitter configured to send information wirelessly to at least one end terminal such as a computer, mobile phone, cloud server, or other hand-held device. Furthermore, the system may comprise a means for transferring a portion of the seminal sample from the macrofluidic fluidic chamber and the microfluidic chip. For example, the system may comprise a pump coupled to the cartridge to pump a portion of the seminal sample into the microfluidic chip.

In a second aspect, the invention relates to a method for analyzing a seminal sample, the method comprising: segregating sperm cells of the seminal sample using a device, detecting the sperm cells in the seminal sample by means of a detecting component, analyzing the sperm cells in the seminal sample by means of an analyzing component, sorting the sperm cells in the seminal sample by means of a sorting component, and recovering the sperm cells from the device.

The method may comprise supplying a flow of the fluidic medium in a medium flow direction, wherein the medium flow direction may be also defined by an initial flow point and a terminal flow point.

The method may comprise introducing the seminal sample into the device at the terminal flow point of the medium flow direction.

The method may comprise recovering sperm cell from the fluidic medium at the initial flow point of the medium flow direction.

The method may further comprise establishing a sorting threshold comprising a sperm profile, wherein the method may comprise sorting the sperm cells based on the sorting threshold.

In one embodiment, analyzing the sperm cells may comprise capturing a first sperm cell image data of the sperm cells, wherein the first sperm cell image data may comprise a first region of the sperm cell, capturing a second sperm cell image date of the sperm cells, wherein the second sperm cell image data may comprise a second region of the sperm cell, and capturing a n-th sperm cell image data of the sperm cells, wherein the n-th sperm cell image data may comprise a n-th region of the sperm cell. Furthermore, analyzing the sperm cells may comprise contrasting each of the sperm image data against a corresponding reference image data.

In one embodiment, capturing the cell image data may be executing automatically using a capturing component. Alternatively or additionally, contrasting each sperm image data against a corresponding reference image data may comprise using a computer-implemented analytical module.

In one embodiment, at least one of the first image data, the second image data, the n-th image data, and the reference image data may comprise at least one video data.

The method may comprise analyzing by means of the computer-implemented analytical module each of the image data and the least one video data via at least one different learning model.

The method may further comprise (automatically) generating a report data set, wherein the report data set may comprise at least one of: sperm concentration, sperm morphology, total motility (MSC: Motile Sperm Concentration), Progressive Motility (PMSC: progressive Motile Sperm Concentration), Sperm DNA Fragmentation.

The method may comprise determining sperm cell quality of the seminal sample, wherein determining the sperm cell quality may comprise determining at least one of: a number of sperm cells in the ejaculate, a percentage of sperm cells with normal morphology, a percentage of motile sperm cells, a percentage of sperm cells with low DNA fragmentation, and a numeric and a visual presentation of the results including at least one representative image data. Additionally or alternatively, such an analysis may be done at population level and/or DNA fragmentation analysis for sperm cells separated as "good quality", i.e. sperms that could swim all the way to the outlet. So, each sperm cell in the subpopulation that was separated in the outlet of the microfluidic device may be analyzed individually for DNA fragmentation.

The method may comprise generating at least one of: a sperm cell quality profile, and a sperm cell quality report.

The recovering of sperm cells from the medium may be performed based on at least one of: a sperm cell quality profile, and a sperm cell quality report. The method may further comprise assessing sperm DNA damage.

In another embodiment, the method may comprise analyzing a birefringence value comprising sperm nuclear integrity levels.

In one embodiment, determining the sperm cell quality may comprise the birefringence value.

The method may further comprise executing a reference analytical procedure, wherein the reference analytical procedure may comprise at least one quality control parameter comprising at least one of sperm dynamic data, sperm DNA integrity, concentration, and morphology. The sperm cells may be mammalian sperm cells.

The method may comprise processing the seminal sample, the processing of the seminal sample comprising detecting health status of the sperm cells.

The method may further comprise using at least one optical device, wherein the health status of the sperm cells may be detected by means of the optical detector. The method may comprise polarizing an optical signal.

The method may comprise at least two variable retarders configured to measure the birefringence of the sperm head

The method may comprise receiving at least one disposable cartridge in the system. The disposable cartridge may be a detachable and replaceable cartridge. The method may further comprise receiving at least one portion of the seminal sample in the device.

The disposable cartridge may comprise a sample carrier, wherein the sample carrier may comprise at least one of: a specimen holding area, and a detachable cover.

In one embodiment, the method may comprise covering the specimen holding area with the detachable cover. In another embodiment, the method may comprise connecting the microfluidic device with the disposable cartridge. Additionally or alternatively, the method may comprise implementing at least one identification mechanism, wherein the method may comprise identifying the disposable cartridge via the at least one identification mechanism. The at least one identification mechanism may comprise using a barcode.

The method may comprise establishing a fluid connection between the microfluidic device and the chamber, wherein the fluid connection may be a permanent fluid connection. In another embodiment, the fluid connection may be a direct fluid connection.

The method selectively establishing a fluid connection between components of the system.

The method may comprise receiving at least one portion of the seminal sample in a detection zone of the device.

The method may comprise analyzing the at least one portion of the seminal sample while the at least one portion of the seminal sample may be contained in the detection zone.

The method may comprise inserting the disposable cartridge in a base component comprising an insertion port. The base component may comprise at least one camera.

The method may comprise measuring at least one signal passing through the seminal sample via the at least one sensor component. The at least one signal may comprise a light signal. The sensor may comprise at least one of: a complementary metal oxide semiconductor (CMOS), a charge-coupled device, a colorimeter, a contact image sensor, an electro-optical sensor, an infrared sensor, a fiber optic sensor, an optical position sensor, a photodetector, a photodiode, a photomultiplier tube, a phototransistor, a photoelectric sensor, a photoionization detector, a photoresistor, a photo-switch, and a photo-tube.

The method may comprise capturing at least one image data of the specimen holding area.

The at least one image data may comprise a single frame image data. And video clip may be at least 5 seconds long.

The at least one image data may comprise a sequence of consecutive individual frames at least 5 frames but less than 660 frames.

The method may comprise capturing at least one first image data by means of a first camera module, and at least one second image data by means of a second camera module

The method may comprise capturing the at least first image data at with the first camera module arranged at a first position, and capturing the at least second image data with the second camera module arranged at a second position, wherein the first position may comprise at least a portion of the first holding area and the second position may comprise at least a portion of the second holding area.

In one embodiment, the method may comprise performing at least one analytical processing on the at least one image data to generate at least one analytical finding. The at least one analytical processing may be performed by means of a main circuit board comprising at least one processor unit configured.

In another embodiment, the method may further comprise performing a first analytical processing on the at least first image data to generate at least one first analytical finding, and a second analytical processing on the at least second image data generate at least one second analytical finding.

In one embodiment, first analytical processing may be different from the second analytical processing. In another embodiment, the first analytical processing and the second analytical processing may comprise an identical analytical processing.

The method may comprise determining a finding with regard to the biological specimen, wherein the finding may be based on at least one the at least one analytical finding. In one embodiment, the finding may be based on the at least first analytical finding. In another embodiment, the finding may be based on the at least second analytical finding. Additionally or alternatively, the finding may be based on the at least first analytical finding and the at least second analytical finding.

The method may comprise transmitting a light signal from at least one light source through the specimen holding area.

The method may further comprise transmitting data from the at least one processor unit to at least one remote device by means of at least one transmitter. Moreover, at least one of the least one remote device may comprise at least one server.

The method may comprise transmitting data comprising information of the seminal sample about at least one of sperm count, sperm morphology, sperm motility, and sperm DNA integrity.

The method may comprise transporting at least a portion of the seminal sample from the macrofluidic chamber into the microfluidic device.

Transporting the at least a portion the seminal sample may be performed by means of at least one pump comprising at least one of: an active pump, and a passive pump.

The method may comprise establishing a fluid connection between at least one of the at least one pump to the specimen holding area.

The method may comprise determining: sperm concentration, sperm morphology, sperm motility, sperm DNA integrity.

The method may further comprise automatically executing auto-calculation by means of an artificial intelligence-based module.

The method may comprise capturing image data with a capture-rate of at least 100 frames per second, at least 660 frames per second, at least 200 frames per second

The method may comprise executing a morphological analysis. The morphological analysis may be based on the captured image data.

The method may further comprise indicating a percentile rank for at least one of: sperm number, sperm morphology, sperm motility, sperm DNA integrity and composite value for the seminal sample.

The method may comprise indicating whether the seminal sample may be considered fertile or sub-fertile.

The sperm motility may comprise an analysis of at least one of: total motility percentage, progressive motility percentage, motile sperm per ejaculate, progressive velocity, mean linearity, motility, viability percentage, and classification of motility as rapid, medium, or slow.

The method may comprise determining at least one threshold value for at least one of: total motility percentage, progressive motility percentage, progressive velocity, motility, and viability.

The sperm morphology may comprise an analyzing at least one of: analysis of head size such small, normal, or large, head shape such as round, tapered, amorphous, midpiece shape and size, tail shape and size, duplicate forms such as multiple heads, multiple tails, and cytoplasmic droplets.

The seminal sample analysis may comprise at least one of: sperm DNA integrity, sex of the sperm, color, viscosity, liquefaction, pH, agglutination, presence of neutrophils, temperature, time since last previous emission, and method of production such as masturbation, coitus interruptus.

The method may comprise establishing a metric for sperm quality base on the sperm DNA integrity comprising at least one measurement of: sperm chromatin organization/architecture, chromatin compaction, and DNA fragmentation.

The method may comprise carrying out any of the preceding method embodiment without utilizing any chemicals for treatment of the samples.

The method may comprise illuminating the seminal sample with a near circular polarized light.

The method may further comprise detecting at least one fiber in at least one direction.

The method may comprise controlling at least liquid crystal device to produce a rapid polarization state.

The method may comprise using Bland-Altman plots of DNA Fragmentation Index (DFI) and High DNA Stainability (HDS) for the seminal samples. The Bland-Altman plots may be based of the seminal sample data, wherein the seminal sample may be treated with Sperm Chromatin Structure Assay (SCSA) and Polarization Microscope.

The method may comprise obtaining the birefringence image representing a retardance and slow axis orientation in every image point of the at least one capture image data.

The method may comprise obtaining at least 1 image data for at least 5 different polarization states. The method may comprise establishing a first polarization setting to produce a circular polarized light.

The method may comprise establishing at least a second polarization setting, wherein the at least second polarization setting yields at least one elliptically polarized light with at least one long axis orientation, wherein each of the least second polarization setting yields at least one long axis orientation different from the circular polarized light and different from the each of the at least one elliptically polarized light.

The method may comprise the at least 5 image data to generate at least one high-resolution map of the seminal sample retardance and a slow axis orientation.

The method may comprise identifying sperm cells in multiple images. Moreover, the method may comprise identifying sperm cells using at least one image feature comprising at least one of: brightness, color, and pixel intensity. In another embodiment, the method may comprise distinguishing sperm cells from debris and other objects in the seminal sample.

The method may comprise tagging individual sperm cells within the at least one image data. The method may comprise encoding individual sperm cells within the at least one image data. The method may comprise using at least one of the tagging and encoding for identification purposes.

The method may comprise comparing the at least one image data with a plurality of multiple sequential image data to track the location of individual sperm cells in the plurality of image data. Furthermore, the method may comprise registering marks or other features in the at least one image, wherein the method may comprise aligning the plurality of images using the marks or other features.

The method may comprise counting sperm cells in the at least one image data, wherein the method may comprise determining the at least one sperm count for the seminal sample.

The method may comprise counting the individual sperm cells in the at least one image data in at least one sequence of image data.

The method may comprise capturing the at least one image data in the detection zone of the microfluidic.

The detection zone may comprise a known volume, wherein the method may comprise determining the number of sperm cells per unit volume based on the known volume.

The method may comprise counting sperm cells only a subset of captured image data. The method may comprise determining the sperm motility for the seminal sample based on information regarding the position of individual sperms in the at least one sequence of image data. The method may comprise comparing the location of individual sperm cells in the at least one sequence of image data. The method may comprise comparing at least two image data in the at least one sequence of image data.

The method may comprise determining the rate and direction of movement of individual sperm cells based on the position. The method may comprise representing movement of individual sperms cells as a vector.

The method may comprise compiling the vector and determining sperm motility for the seminal sample. Moreover, the method may comprise tracking the number of individual sperm cells and the number of image data in the at least one sequence of image data.

The invention also relates to a use of the system as recited herein for carrying out the method as recited herein
The present technology is also defined by the following numbered embodiments.

Below, system embodiments will be discussed. These embodiments are abbreviated by the letter "S" followed by a number. When reference is herein made to a system embodiment, those embodiments are meant.
S1. A system for processing a seminal sample, the system comprising
   a detecting component configured to detect sperm cells in the seminal sample,
   an analyzing component configured to analyze sperm cells in the seminal sample, and
   a sorting component configured to sort sperm cells in the seminal sample.
S2. The system according to the preceding embodiment, wherein the system is configured to detect health status of the sperm cells.
S3. The system according to any of the 2 preceding embodiments, wherein the system further comprises at least one optical device.
S4. The system according to the preceding embodiment, wherein the system comprises at least one linear polarizer.
S5. The system according to any of the 3 preceding embodiments, wherein the system comprises at least two variable retarders.
S6. The system according to any of the 4 preceding embodiments, wherein the system comprises at least one light source.
S7. The system according to any of the preceding system embodiments, wherein the system is configured to receive a disposable cartridge.
S8. The system according to the preceding embodiment, wherein the disposable cartridge is detachable and replaceable cartridge.
S9. The system according to any of the preceding system embodiments, wherein the system comprises a base component.
S10. The system according to any of the preceding system embodiments, wherein the system further comprises a microfluidic device configured to receive at least one portion of the seminal sample.
511. The system according to the preceding embodiment, wherein the disposable cartridge comprises a sample carrier, wherein the sample carrier comprises a specimen holding area.
S12. The system according to the preceding embodiment, wherein the specimen holding area comprises a top side and a bottom side, wherein the top side is opposite to the bottom side.
S13. The system according to the preceding embodiment, wherein the specimen holding area comprises a detachable cover configured to cover the specimen holding area, wherein detachable cover is arranged on the top side of the specimen holding area.
S14. The system according to any of the preceding system embodiments, wherein the disposable cartridge also may comprise a microfluidic element fluidly connected with the disposable cartridge.
S15. The system according to any of the preceding system embodiments and with features of embodiment S7, wherein the disposable cartridge comprises at least one identification mechanism.
S16. The system according to the preceding embodiment, wherein the at least one identification mechanism comprises a barcode.
S17. The system according to any of the preceding system embodiments, wherein the system comprises a fluid connection between the microfluidic device and the chamber.
S18. The system according to the preceding embodiment, wherein the fluid connection is a permanent fluid connection.
S19. The system according to the 2 preceding embodiments, wherein the fluid connection is a direct fluid connection.
S19. The system according to embodiment S17, wherein the system comprise at least one valve arranged between the microfluidic device and the chamber, wherein the at least one valve is configured to selectively assume at least one of at least two operational states
S20. The system according to the preceding embodiment, wherein the at least two operational states comprises a first operational state and a second, wherein the system is configured to establish a fluid connection between the microfluidic element and the disposable cartridge in the first operational state, and to discontinue the fluidic connection between the microfluidic element and the disposable cartridge while in the second operational state.
S21. The system according to any of the preceding system embodiments and with features of embodiment S10, wherein the microfluidic device may comprise a detection zone configured to receive the at least one portion of the seminal sample.
S22. The system according to the preceding embodiment, wherein the system is configured to analyzed the at least one portion of the seminal sample while the at least one portion of the seminal sample is contained in the detection zone.
S23. The system according to any of the preceding system embodiments and with features of embodiment S9, wherein the base component comprises an insertion port configured to allow inserting the disposable microfluidic device into the base component.
S24. The system according to any of the preceding system embodiments and with features of embodiment S9, wherein the base component comprises at least one a camera.
S25. The system according to any of the preceding system embodiments and with features of embodiment S3, wherein the at least one optical device comprises at least one lens system.
S26. The system according to any of the preceding system embodiments with features of embodiments S3, wherein the at least one optical device comprises a proximal section and a distal section.
S27. The system according to the preceding embodiment and with features of embodiments S25, wherein the at least one lens system is arranged in a portion of the proximal section of the at least one optical device. 4
S28. The system according to any of the preceding system embodiments, wherein at least one lens system comprises a magnifying lens.
S29. The system according to the preceding embodiment, wherein the magnifying lens is a microscope lens.
S30. The system according to any of the preceding system embodiments and with features of embodiment S25, wherein the at least one lens system further comprises a central magnifying lens and an opaque material, wherein the opaque material surrounds the central magnifying lens.
S31. The system according to any of the preceding system embodiments and with features of embodiment S3, wherein at least one optical device comprises at least one sensor component arranged on specimen holding at a side of the at least one lens system.
S32. The system according to the preceding embodiment, wherein the at least one sensor component is configured to measure at least one signal passing through the seminal sample.
S33. The system according to the preceding embodiment, wherein the at least one signal comprises a light signal, wherein the light signal passes from the seminal sample through the at least one lens system.
S34. The system according to any of the preceding embodiments and with features of embodiment S31, wherein the sensor comprises at least one of: a complementary metal oxide semiconductor (CMOS), a charge-coupled device, a colorimeter, a contact image sensor, an electro-optical sensor, an infrared sensor, a fiber optic sensor, an optical position sensor, a photodetector, a photodiode, a photomultiplier tube, a phototransistor, a photoelectric sensor, a photoionization detector, a photoresistor, a photo-switch, and a photo-tube.
S35. The system according to any of the preceding, wherein the at least one camera is configured to capture at least one image data of the specimen holding area.
S36. The system according to the pre ceding embodiment, wherein the at least one image data comprises a single frame image data.
S37. The system according to any of the 2 preceding embodiments, wherein the at least one image data comprises a sequence of consecutive individual frames comprising an image capture of at least 10 s⁻¹, preferably at least 90 s⁻¹, more preferably at least 300 s⁻¹, such as at least 600 s⁻¹.
S38. The system according to any of the preceding embodiments and with features of embodiment S35, wherein the system comprises at least two camera modules, wherein the system comprises a first camera module configured to capture at least one first image data, and a second camera module configured to capture at least one second image.
S39. The system according to the preceding embodiment, wherein the first camera module is arranged at a first position and the second camera module is arranged at a second position, wherein the first position is different from the second position.
S39. The system according to the 2 preceding embodiments, wherein the first camera module is configured to capture the at least first image data at the first position and the second camera module is configured to capture the at least second image data at the second position, wherein the first position comprises at least a portion of the first holding area and the second position comprises at least a portion of the second holding area.
S40. The system according to any of the preceding embodiments and with features of embodiment S35, wherein the system comprises a main circuit board comprising at least one processor unit configured to perform at least one analytical processing on the at least one image data to generate at least one analytical finding.
S41. The system according to the preceding embodiment, wherein the at least one processor unit is configured to perform
   a first analytical processing on the at least first image data to generate at least one first analytical finding, and
   a second analytical processing on the at least second image data generate at least one second analytical finding.
S42. The system according to the preceding embodiment, wherein the first analytical processing is different from the second analytical processing.
S43. The system according to embodiment S41, wherein the first analytical processing and the second analytical processing comprises an identical analytical processing.
S44. The system according to any of the preceding system embodiments and with features of embodiment S40, wherein the at least one processor unit is configured to determine a finding with regard to the biological specimen, wherein the finding is based on at least one the at least one analytical finding.
S45. The system according to the preceding embodiment, wherein the finding is based on the at least first analytical finding
S46. The system according to embodiment S44, wherein the finding is based on the at least second analytical finding.
S47. The system according to the 3 preceding embodiments, wherein the finding is based on the at least first analytical finding and the at least second analytical finding.
S48. The system according to any of the preceding system embodiments, wherein the system comprises a housing configured to enclose at least one of: the at least one processor unit, the main circuit board, and at least one of the at least two cameras.
S49. The system according to any of the preceding system embodiments and with features of embodiment S38, wherein at least one of the at least two cameras comprises a polarizer configured to facilitate birefringence measurement of the specimen.
S50. The system according to any of the preceding system embodiments, wherein the system comprises at least one lighting system, wherein at least one of the least one lighting system comprises at least one light source.
S51. The system according to the preceding, wherein the lighting system comprises optical fibers configured to transmit light from the at least one light source to the specimen holding area.
S52. The system according to the preceding, wherein the system comprises at least one transmitter configured to transmit data from the at least one processor unit to at least one remote device.
S53. The system according to the preceding embodiment, wherein at least one of the least one remote device comprises at least one server.
S54. The system according to any of the 2 preceding embodiments, wherein the at least one transmitter is configured to transmit data comprising information of the seminal sample about at least one of: sperm count, sperm morphology, sperm motility, and sperm DNA integrity.
S55. The system according to any of the preceding system embodiments, wherein the system comprises at least one pump configured to transport at least a portion of the seminal sample from the macrofluidic fluidic chamber into the microfluidic device.
S56. The system according to the preceding embodiment, wherein at least one of the least one pump is an active pump.
S57. The system according to any of the 2 preceding embodiments, wherein at least of the least one pump is a passive pump.
S58. The system according to any of the 3 preceding embodiments, wherein at least one of the at least one pump is fluidly connected to the specimen holding area.
S59. The system according to any of the preceding system embodiments, wherein the system is further configured to select sperm cells based on the sperm cells health status.
S60. The system according to any of the preceding system embodiments, wherein the system is also configured to determine sperm concentration.
S61. The system according to any of the preceding system embodiments, wherein the system is further configured to determine sperm motility.
S62. The system according to any of the preceding system embodiments, wherein the system is further configured to determine sperm DNA integrity.
S63. The system according to any of the preceding system embodiments, wherein the system comprises a built-in high-resolution and autofocus optical lens.
S64. The system according any of the preceding system embodiments, wherein the system further comprises an artificial intelligence-based module configured to execute auto-calculations.
S65. The system according to any of the preceding system embodiments, wherein the autofocus optical lens is configured to capture image data with a capture-rate of at least 60 frames per second, at least 100 frames per second, at least 660 frames per second, at least 200 frames per second
S66. The system according to any of the preceding system embodiments, wherein the system is configured to execute a morphological analysis.
S67. The system according to any of the preceding system embodiments, wherein the morphological analysis is based on the captured image data.
S68. The system according to any of the preceding system embodiments, wherein the at least one processor unit comprised by the analytical device is configured to receive optical data from the optical device.
S69. The system according to any of the preceding system embodiments, wherein the at least one processor unit is configured to perform a high-level, single-thread object detection and/or tracking.
S70. The system according to any of the preceding system embodiments, wherein the at least one processor unit is single-core unit.
S71. The system according to any of the preceding system embodiments, wherein the at least one processor unit is a multi-core unit.
S72. The system according to any of the preceding system embodiments, wherein the at least one processor unit comprises a speed of less than about 5 GHz, less than 3 GHz, less than 3 GHz, less than 500 MHz, such as, for example, or less than 250 MHz.
S73. The system according to any of the preceding system embodiments, wherein the analytical device comprises a power source.
S74. The system according to the preceding system embodiment, wherein the power source is a battery. In one embodiment, the power source is a battery.
S75. The system according to any of the preceding system embodiments, wherein the analytical device comprises a lighting system comprising a light source suitable for microscopy, such as a tungsten lamp, halogen lamp, mercury lamp, xenon lamp, or LED lamp.
S76. The system according to the preceding system embodiments, wherein the lighting system comprises optical fibers configured to transmit light from the light source to the receptacle.
S77. The system according to the 2 preceding system embodiments, wherein the optical fibers is configured to focus the light on the microfilament element or on the detection zone of the microfilament.
S78. The system according to any of the 3 preceding system embodiments, wherein lighting system is configured to illuminate the microfluidic device, wherein the microfluid device comprises at one layer of a reflective coating on the walls of the microfluidic device, wherein the at least one layer is configured to focus the light on the detection zone of the microfluidic device.

Below, method embodiments will be discussed. These embodiments are abbreviated by the letter "M" followed by a number. When reference is herein made to a method embodiment, those embodiments are meant.
M1. A method for analyzing a seminal sample, the method comprising
   segregating sperm cells of the seminal sample using a device,
   detecting the sperm cells in the seminal sample by means of a detecting component, analyzing the sperm cells in the seminal sample by means of an analyzing component,
   sorting the sperm cells in the seminal sample by means of a sorting component, and recovering the sperm cells from the device.
M2. The method according to the preceding embodiment, wherein the method comprises supplying a flow of the fluidic medium in a medium flow direction, wherein the medium flow direction is also defined by an initial flow point and a terminal flow point.
M3. The method according to the 2 preceding embodiments, wherein the method comprises introducing the seminal sample into the device at the terminal flow point of the medium flow direction.
M4. The method according to any of the 2 preceding embodiments, wherein the method comprises recovering sperm cell from the fluidic medium at the initial flow point of the medium flow direction.
M5. The method according to any of the preceding method embodiments, wherein the method further comprises establishing a sorting threshold comprising a sperm profile, wherein the method comprises sorting the sperm cells based on the sorting threshold.
M6. The method according to the preceding embodiment, wherein analyzing the sperm cells comprises
   capturing a first sperm cell image data of the sperm cells, wherein the first sperm cell image data comprises a first region of the sperm cell,
   capturing a second sperm cell image date of the sperm cells, wherein the second sperm cell image data comprises a second region of the sperm cell, and
   capturing a n-th sperm cell image data of the sperm cells, wherein the n-th sperm cell image data comprises a n-th region of the sperm cell.
M7. The method according to the preceding embodiment, wherein analyzing the sperm cells further comprises contrasting each of the sperm image data against a corresponding reference image data.
M8. The method according to the 2 preceding embodiments, wherein capturing the cell image data is executing automatically using a capturing component.
M9. The method according to the 3 preceding embodiments, wherein contrasting each sperm image data against a corresponding reference image data comprises using a computer-implemented analytical module.
M10. The method according to any of the preceding method embodiments, wherein at least one of: the image first image data, the second image data, the n-th image data, and the reference image data comprises at least one video data.
M11. The method according to the 2 preceding embodiments, wherein the method comprises analyzing by means of the computer-implemented analytical module each of the image data and the least one video data via at least one different learning model.
M12. The method according to any of the preceding method embodiments, wherein the method further comprises (automatically) generating a report data set, wherein the report data set comprises at least one of: sperm concentration, sperm morphology, total motility (MSC: Motile Sperm Concentration), Progressive Motility (PMSC: progressive Motile Sperm Concentration), Sperm DNA Fragmentation.
M13. The method according to any of the preceding method embodiments, wherein the method comprises determining sperm cell quality of the seminal sample, wherein determining the sperm cell quality comprises determining at least one of: a number of sperm cells in the ejaculate, a percentage of sperm cells with normal morphology, a percentage of motile sperm cells, a percentage of sperm cells with low DNA fragmentation, and a numeric and a visual presentation of the results including at least one representative image data.
M14. The method according to the preceding embodiment, wherein the method comprises generating at least one of: a sperm cell quality profile, and a sperm cell quality report.
M15. The method according to the 2 preceding embodiments, wherein the recovering of sperm cells from the medium is performed based on at least one of: a sperm cell quality profile, and a sperm cell quality report.
M16. The method according to any of the preceding embodiment, wherein the method further comprises assessing sperm DNA damage.
M17. The method according to any of the preceding method embodiments, wherein the method comprises analyzing a birefringence value comprising sperm nuclear integrity levels.
M19. The method according to the preceding embodiment, wherein the step of determining the sperm cell quality comprises the birefringence value.
M20. The method according to any of the preceding method embodiments, wherein the method further comprises executing a reference analytical procedure, wherein the reference analytical procedure comprises at least one quality control parameter comprising at least one of: sperm dynamic data, sperm DNA integrity, concentration, and morphology.
M21. The method according to any of the preceding method embodiments, wherein the sperm cells are mammalian sperm cells.
M22. The method according to any of the preceding method embodiments, wherein the method comprises processing the seminal sample, the processing of the seminal sample comprising detecting health status of the sperm cells.
M23. The method according to any of the 2 preceding embodiments, wherein the method further comprises using at least one optical device, wherein the health status of the sperm cells is detected by means of the optical detector.
M24. The method according to the preceding embodiment, wherein the method comprises polarizing an optical signal.
M25. The method according to any of the 3 preceding embodiments, wherein the method comprises at least two variable retarders configured to measure the birefringence of the sperm head
M26. The method according to any of the preceding method embodiments, wherein the method comprises receiving at least one disposable cartridge in the system.
M27. The method according to the preceding embodiment, wherein the disposable cartridge is a detachable and replaceable cartridge.
M28. The method according to any of the preceding method embodiments, wherein the method further comprises receiving at least one portion of the seminal sample in the device.
M29. The method according to the preceding embodiment, wherein the disposable cartridge comprises a sample carrier, wherein the sample carrier comprises at least one of: a specimen holding area, and a detachable cover.
M30. The method according to the preceding embodiment, wherein the method comprises covering the specimen holding area with the detachable cover.
M31. The method according to any of the preceding method embodiments, wherein the method comprises connecting the microfluidic device with the disposable cartridge.
M32. The method according to any of the preceding method embodiments, wherein the method comprises implementing at least one identification mechanism, wherein the method comprises identifying the disposable cartridge via the at least one identification mechanism.
M33. The method according to the preceding embodiment, wherein the at least one identification mechanism comprises using a barcode.
M34. The method according to any of the preceding method embodiments, wherein the method comprises establishing a fluid connection between the microfluidic device and the chamber.
M35. The method according to the preceding embodiment, wherein the fluid connection is a permanent fluid connection.
M36. The method according to the 2 preceding embodiments, wherein the fluid connection is a direct fluid connection.
M37. The method according to embodiment M34, wherein the method selectively establishing a fluid connection between components of the system.
M38. The method according to any of the preceding method embodiments and with features of embodiment xx, wherein the method comprises receiving at least one portion of the seminal sample in a detection zone of the device.
M39. The method according to the preceding embodiment, wherein the method comprises analyzing the at least one portion of the seminal sample while the at least one portion of the seminal sample is contained in the detection zone.
M40. The method according to any of the preceding method embodiments, wherein the method comprises inserting the disposable cartridge in a base component comprising an insertion port.
M41. The method according to any of the preceding method embodiments, wherein the base component comprises at least one camera.
M42. The method according to the preceding embodiment, wherein the method comprises measuring at least one signal passing through the seminal sample via the at least one sensor component.
M43. The method according to the preceding embodiment, wherein the at least one signal comprises a light signal.
M44. The method according to any of the preceding method embodiments, wherein the sensor comprises at least one of: a complementary metal oxide semiconductor (CMOS), a charge-coupled device, a colorimeter, a contact image sensor, an electro-optical sensor, an infrared sensor, a fiber optic sensor, an optical position sensor, a photodetector, a photodiode, a photomultiplier tube, a phototransistor, a photoelectric sensor, a photoionization detector, a photoresistor, a photo-switch, and a photo-tube.
M45. The method according to any of the preceding, wherein the method comprises capturing at least one image data of the specimen holding area.
M46. The method according to the pre ceding embodiment, wherein the at least one image data comprises a single frame image data.
M47. The method according to any of the 2 preceding embodiments, wherein the at least one image data comprises a sequence of consecutive individual frames at least 5 frames but less than 660 frames.
M48. The method according to any of the preceding method embodiments, wherein the method comprises capturing: at least one first image data by means of a first camera module, and at least one second image data by means of a second camera module
M49. The method according to the 2 preceding embodiments, wherein the method comprises
   capturing the at least first image data at with the first camera module arranged at a first position, and
   capturing the at least second image data with the second camera module arranged at a second position, wherein the first position comprises at least a portion of the first holding area and the second position comprises at least a portion of the second holding area.
M50. The method according to any of the preceding method embodiments, wherein the method comprises performing at least one analytical processing on the at least one image data to generate at least one analytical finding.
M51. The method according to the preceding embodiments, wherein the at least one analytical processing is performed by means of a main circuit board comprising at least one processor unit configured.
M52. The method according to the preceding embodiment, wherein the method further comprises performing
   a first analytical processing on the at least first image data to generate at least one first analytical finding, and
   a second analytical processing on the at least second image data generate at least one second analytical finding.
M53. The method according to the preceding embodiment, wherein the first analytical processing is different from the second analytical processing.
M54. The method according to embodiment M52, wherein the first analytical processing and the second analytical processing comprises an identical analytical processing.
M55. The method according to any of the preceding method embodiments, wherein the method comprises determining a finding with regard to the biological specimen, wherein the finding is based on at least one the at least one analytical finding.
M56. The method according to the preceding embodiment, wherein the finding is based on the at least first analytical finding.
M57. The method according to embodiment M45, wherein the finding is based on the at least second analytical finding.
M58. The method according to the 3 preceding embodiments, wherein the finding is based on the at least first analytical finding and the at least second analytical finding.
M59. The method according to the preceding embodiment, wherein the method comprises transmitting a light signal from at least one light source through the specimen holding area.
M60. The method according to the preceding embodiment, wherein the method further comprises transmitting data from the at least one processor unit to at least one remote device by means of at least one transmitter.
M61. The method according to the preceding embodiment, wherein at least one of the least one remote device comprises at least one server.
M62. The method according to any of the 2 preceding embodiments, wherein the method comprises transmitting data comprising information of the seminal sample about at least one of: sperm count, sperm morphology, sperm motility, and sperm DNA integrity.
M63. The method according to any of the preceding method embodiments, wherein the method comprises transporting at least a portion of the seminal sample from the macrofluidic chamber into the microfluidic device.
M64. The method according to the preceding embodiment, wherein transporting the at least a portion the seminal sample is performed by means of at least one pump comprising at least one of: an active pump, and a passive pump.
M65. The method according to any of the 3 preceding embodiments, wherein the method comprises establishing a fluid connection between at least one of the at least one pump to the specimen holding area.
M66. The method according to any of the preceding method embodiments, wherein the method comprises determining sperm concentration.
M67. The method according to any of the preceding method embodiments, wherein the method comprises determining sperm morphology.
M68. The method according to any of the preceding method embodiments, wherein the method comprises determining sperm motility.
M69. The method according to any of the preceding method embodiments, wherein the method comprises determining sperm DNA integrity.
M70. The method according to any of the preceding method embodiments, wherein the method further comprises automatically executing auto-calculation by means of an artificial intelligence-based module.
M71. The method according to any of the preceding method embodiments, wherein the method comprises capturing image data with a capture-rate of at least 100 frames per second, at least 660 frames per second, at least 200 frames per second
M72. The method according to any of the preceding method embodiments, wherein the method comprises executing a morphological analysis.
M73. The method according to any of the preceding method embodiments, wherein the morphological analysis is based on the captured image data.
M74. The method according to any of the preceding method, wherein the method further comprises indicating a percentile rank for at least one of: sperm number, sperm morphology, sperm motility, sperm DNA integrity and composite value for the seminal sample.
M75. The method according to the preceding embodiment, wherein the method comprises indicating whether the seminal sample is considered fertile or sub-fertile.
M76. The method according to the 2 preceding embodiments, wherein the sperm motility may comprise an analysis of at least one of: total motility percentage, progressive motility percentage, motile sperm per ejaculate, progressive velocity, mean linearity, motility, viability percentage, and classification of motility as rapid, medium, or slow.
M77. The method according to any of the preceding method embodiments, wherein the method comprises determining at least one threshold value for at least one of: total motility percentage, progressive motility percentage, progressive velocity, motility, and viability.
M78. The method according to any of the preceding method, wherein the sperm morphology comprises an analyzing at least one of: analysis of head size such small, normal, or large, head shape such as round, tapered, amorphous, midpiece shape and size, tail shape and size, duplicate forms such as multiple heads, multiple tails, and cytoplasmic droplets.
M79. The method according to any of the preceding method embodiments, wherein the seminal sample analysis comprises at least one of: sperm DNA integrity, sex of the sperm, color, viscosity, liquefaction, pH, agglutination, presence of neutrophils, temperature, time since last previous emission, and method of production such as masturbation, coitus interruptus.
M80. The method according to any of the preceding method embodiments, wherein the method comprises establishing a metric for sperm quality base on the sperm DNA integrity comprising at least one measurement of: sperm chromatin organization/architecture, chromatin compaction, and DNA fragmentation.
M81. The method according to any of the preceding method embodiments, wherein the method comprises carrying out any of the preceding method embodiment without utilizing any chemicals for treatment of the samples.
M82. The method according to any of the preceding method embodiments, wherein the method comprises illuminating the seminal sample with a near circular polarized light.
M83. The method according to any of the preceding method embodiments, wherein the method further comprises detecting at least one fiber in at least one direction.
M84. The method according to any of the preceding method embodiments, wherein the method comprises controlling at least liquid crystal device to produce a rapid polarization state.
M85. The method according to any of the preceding method embodiments, wherein the method comprises using Bland-Altman plots of DNA Fragmentation Index (DFI) and High DNA Stainability (HDS) for the seminal samples.
M86. Th method according to the preceding embodiment, wherein the Bland-Altman plots are based on the seminal sample data, wherein the seminal sample is treated with sperm chromatin structure assay (SCSA) and Polarization Microscope.
M87. The method according to any of the preceding method embodiments, wherein the method comprises obtaining the birefringence image representing a retardance and slow axis orientation in every image point of the at least one capture image data.
M88. The method according to the preceding embodiment, wherein the method comprises obtaining at least 1 image data for at least 5 different polarization states.
M89. The method according to the 2 preceding embodiments, wherein the method comprises establishing a first polarization setting to produce a circular polarized light. M90. The method according to the 3 preceding embodiments, wherein the method comprises establishing at least a second polarization setting, wherein the at least second polarization setting yields at least one elliptically polarized light with at least one long axis orientation, wherein each of the least second polarization setting yields at least one long axis orientation different from the circular polarized light and different from the each of the at least one elliptically polarized light.
M91. The method according to any of the preceding method embodiments, wherein the method comprises the at least 5 image data to generate at least one high-resolution map of the seminal sample retardance and a slow axis orientation.
M92. The method according to any of the preceding method embodiments, wherein the method comprises identifying sperm cells in multiple images.
M93. The method according to the preceding embodiment, wherein the method comprises identifying sperm cells using at least one image feature comprising at least one of: brightness, color, and pixel intensity.
M94. The method according to the 2 preceding embodiments, wherein the method comprises distinguishing sperm cells from debris and other objects in the seminal sample.
M95. The method according to any of the preceding method embodiments, wherein the method comprises tagging individual sperm cells within the at least one image data.
M96. The method according to any of the preceding method embodiments, wherein the method comprises encoding individual sperm cells within the at least one image data.
M97. The method according to any of the 2 preceding embodiments, wherein the method comprises using at least one of the tagging and encoding for identification purposes.
M98. The method according to any of the preceding method embodiments, wherein the method comprises comparing the at least one image data with a plurality of multiple sequential image data to track the location of individual sperm cells in the plurality of image data.
M99. The method according to any of the preceding method embodiments, wherein the method comprises registering marks or other features in the at least one image, wherein the comprises aligning the plurality of images using the marks or other features.
M100. The method according to any of the preceding method embodiments, wherein the method comprises counting sperm cells in the at least one image data, wherein the method comprises determining the at least one sperm count for the seminal sample.
M101. The method according to the preceding method embodiment, wherein the method comprises counting the individual sperm cells in the at least one image data in at least one sequence of image data.
M102. The method according to any of the 2 preceding embodiments, wherein the method comprises capturing the at least one image data in the detection zone of the microfluidic.
M103. The method according to any of the preceding method embodiments, wherein detection zone comprises a known volume, wherein the method comprises determining the number of sperm cells per unit volume based on the known volume.
M104. The method according to any of the preceding method embodiments, wherein the method comprises counting sperm cells only a subset of captured image data.
M105. The method according to any of the preceding method embodiments, wherein the method comprises determining the sperm motility for the seminal sample based on information regarding the position of individual sperms in the at least one sequence of image data.
M106. The method according to the preceding embodiment, wherein the method comprises comparing the location of individual sperm cells in the at least one sequence of image data.
M107. The method according to the preceding embodiment, wherein the method comprises comparing at least two image data in the at least one sequence of image data.
M108. The method according to 3 the preceding embodiments, wherein the method comprises determining the rate and direction of movement of individual sperm cells based on the position.
M109. The method according to 4 the preceding embodiments, wherein the method comprises representing movement of individual sperms cells as a vector.
M110. The method according to the preceding embodiment, wherein the method comprises compiling the vector and determining sperm motility for the seminal sample.
M111. The method according to any of the preceding method embodiments, wherein the method comprises tracking the number of individual sperm cells and the number of image data in the at least one sequence of image data.
U1. Use of the system according to any of the preceding system embodiments for carrying out the method according to any of the preceding method embodiments.

The present invention will now be described with reference to the accompanying drawings which illustrate embodiments of the invention. These embodiments should only exemplify, but not limit, the present invention.
- Fig. 1: depicts a system for analyzing semen samples according to embodiments of the present invention;
- Fig. 2: schematically depicts an imaging workflow carried out in a system according to embodiments of the present invention;
- Fig. 3: depicts a Bland-Altman plot for DFI (left) and HDS (right) in human sperm;
- Fig. 4: depicts a Bland-Altman plot for DFI (left) and HDS (right) in bull sperm.

It is noted that not all the drawings carry all the reference signs. Instead, in some of the drawings, some of the reference signs have been omitted for sake of brevity and simplicity of illustration. Embodiments of the present invention will now be described with reference to the accompanying drawings.

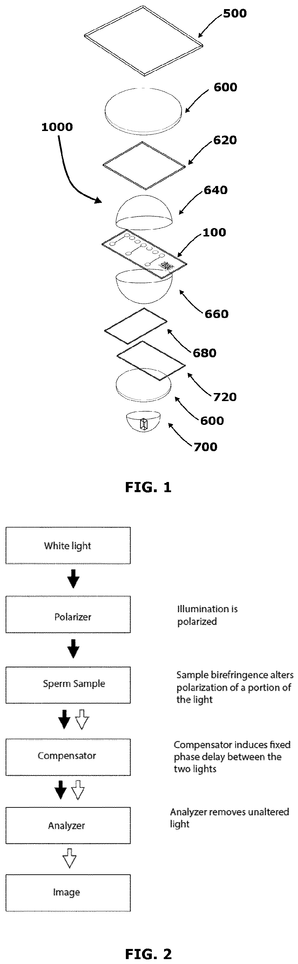

Fig. 1 schematically depicts a system 1000 for analyzing semen samples according to embodiments of the present invention. In simple terms, the system may comprise may comprise at least one optical device 500, at least one linear polarizer 600, a quarter wave plate 620, an objective 640, a condenser 660, a LC-B variable retarder 680, a LC-A variable retarder 720, at least one linear polarizer and a light source 700.

In one embodiment, the system may also comprise a disposable cartridge, a microfluidic chip and a base component. The disposable cartridge may also be referred to as macrofluidic chamber. The disposable cartridge may comprise a sample carrier comprising a specimen holding area and/or a detachable cover. The specimen holding area may also comprise a top side and a bottom side, which may also be simply be referred to as top and bottom, respectively. In one embodiment, the detachable cover may be arranged on the top side of the specimen holding area. It should be understood that the microfluidic chip may be configured for biological specimen.

Moreover, the disposable cartridge also may comprise a microfluidic element fluidly connected with the disposable cartridge. The disposable cartridge may comprise a barcode for sample identification.

The microfluidic element may be configured to receive a portion of the semen sample. Furthermore, the fluidic connection between the microfluidic element and the chamber may comprise a permanent direct fluid connection between the microfluidic element and the disposable cartridge. In another embodiment, the microfluidic element and the chamber may be separated by a valve or similar unit that may allow to selectively control the fluidic connection: in more simple words, the valve may comprise two operational states different from each other, wherein a first operational state may allow to establish a fluid connection between the microfluidic element and the disposable cartridge, i.e. the valve may be "open", and a second operational state may allow to discontinue the fluidic connection between the microfluidic element and the disposable cartridge, i.e. the valve may be "closed".

Additionally or alternatively, the microfluidic element may comprise a detection zone in which a portion of the semen sample can be analyzed by means of the analytical device. The wall of the microfluidic element situated between the detection zone and the analytical device is transparent so that light can pass from the portion of the sample in the detection zone into the optical device. The detection zone is dimensioned to accommodate a known volume so that the number of sperm cells counted in a portion of the detection zone can be extrapolated to determine the sperm number of the semen sample. As used herein, the sperm number of a semen sample refers to the number of sperm cells per unit of volume of semen and is used interchangeably with sperm count and sperm/semen density. The detection zone may comprise one or more reference objects of known dimensions and/or position to allow scaling and registration of images captured by the optical device.

Additionally or alternatively, the base component may comprise an insertion port and/or a camera. The insertion port may be configured for inserting the disposable microfluidic device into the base component.

In one embodiment, the optical device may comprise a lens system. The at least one lens system may be in a portion of the optical device proximal to the receptacle. The at least one lens system may comprise a magnifying lens, such as a microscope lens. The at least one lens system may comprise a central magnifying lens surrounded by an opaque material. At least one optical device may comprise a sensor on the receptacle-distal side of the at least one lens system. The sensor senses light signals that pass from the semen sample through the at least one lens system. The sensor may be a complementary metal oxide semiconductor (CMOS), charge-coupled device, colorimeter, contact image sensor, electro-optical sensor, infrared sensor, fiber optic sensor, optical position sensor, photodetector, photodiode, photomultiplier tubes, phototransistor, photoelectric sensor, photoionization detector, photomultiplier, photoresistor, photo switch, or phototube. The camera captures one or more video clip(s) and image(s) of the specimen holding area.

In one embodiment, the system may comprise two camera modules, wherein a first camera module may be arranged at a first position to capture at least one first image data comprising the first holding area, and a second camera module may be arranged at a second position to capture at least one second image data of the second holding area. It should be understood that the at least one image data may comprise a plurality of single independent image frames and/or a sequence of consecutive frames comprising a frame rate, e.g. a videoclip.

In another embodiment, the system may also comprise a main circuit board comprising at least one processor unit configured to perform a first analytic processing of the at least one image data of the first holding area to generate a first analytic finding. In a further embodiment, the processor may also be configured to perform a second analytic processing of the at least one second image data of the second holding area to generate a second analytic finding. In one embodiment, the second analytic processing may be different from the first analytic processing.

Additionally or alternatively, the at least one processor unit may also be configured to determine an outcome with regard to the biological specimen, wherein the outcome is based on at least one the first analytic finding and/or the second analytic finding.

In one embodiment, the system may also comprise a housing, which also be referred to as a casing. The housing may be configured to enclose at least of the at least one processor unit, the main circuit board, and at least one of the two cameras 500.

Furthermore, in one embodiment at least one of the two cameras 500 may comprise a polarizer configured to facilitate birefringence measurement of the specimen, e.g. the second camera may be equipped with a polarizer for birefringence measurement of the specimen.

The analytical device may comprise a lighting system. The lighting system may comprise a light source. The lighting system may comprise optical fibers configured to transmit light from the light source to the specimen holding area.

The analytical device may comprise a transmitter configured to configure to transmit data from the at least one processor unit to a remote device. The transmitter may be configured to transmit data comprising information about at least one of sperm count, sperm morphology, sperm motility, and sperm DNA integrity of the semen sample.

The system may comprise a pump configured to configured to transport at least a portion of the semen sample from the macrofluidic fluidic chamber into the microfluidic element. In one embodiment, the pump may be an active pump. In another embodiment, the pump may be a passive pump. Additionally or alternatively, the pump may be coupled to the receptacle.

The analytical device may comprise a power source. In one embodiment, the power source may be a battery.

Moreover, the analytical device may comprise a lighting system comprising a light source suitable for microscopy, such as a tungsten lamp, halogen lamp, mercury lamp, xenon lamp, or LED lamp.

In one embodiment, the lighting system may comprise optical fibers configured to transmit light from the light source to the receptacle. The optical fibers may focus the light on the microfilament element or on the detection zone of the microfilament. In another embodiment, the lighting system may be configured to illuminate the microfluidic chip, wherein the microfluid chip may comprise at one layer of a reflective coating on the walls of the microfluidic chip, wherein the at least one layer may be configured to focus the light on the detection zone of the microfluidic element.

The analytical device may comprise a transmitter configured to transmit data comprising information about at least one of sperm number, sperm morphology and sperm motility of the semen sample to a remote device. For example, the analytical device may comprise a radio frequency transmitter configured to send information wirelessly to at least one end terminal such as a computer, mobile phone, or other hand-held device.

Fig. 2 schematically depicts an imaging workflow carried out in the system 1000. In the context of the analytical method, a sperm count, sperm morphology, and/or sperm motility of semen samples may be analyzed by reference to a standard, such as the reference values adopted by the World Health Organization. WHO laboratory manual for the examination and processing of human semen, Fifth Edition, 2010, incorporated herein by reference. Under WHO standards, a semen sample is considered normal if the data for the sample is at or above the 5th percentile. According to the WHO 2010 standards, threshold values for sperm number, sperm morphology, and sperm motility are 15 million/ml, 4% normal forms, and 32% progressive motility, respectively. Thus, analysis of samples may indicate a percentile rank for sperm number, sperm morphology, and sperm motility individually and/or a composite value for the semen sample. Furthermore, analysis of samples may also include an indication of whether the sample is considered fertile or sub-fertile.

Moreover, analysis of sperm motility may comprise analysis of total motility percentage, progressive motility percentage, motile sperm per ejaculate, progressive velocity, mean linearity, motility index, viability percentage, or classification of motility as rapid, medium, or slow. Any of these values may be analyzed by reference to a standard. For example, according to WHO 2010 standards, threshold values for the following parameters are as follows: total motility percentage, 40%; progressive motility percentage, 32%; progressive velocity, 25µm is; motility index, 1 O; and viability, 58%.

Additionally or alternatively, analysis of sperm morphology may comprise analysis of head size (e.g., small, normal, or large), head shape (e.g., round, tapered, amorphous), midpiece shape and size; tail shape and size; duplicate forms (multiple heads, multiple tails), or cytoplasmic droplets. Any of these values may be analyzed by reference to a standard.

The analysis of a semen sample may comprise analysis of the sample volume. Analysis of sample volume may also include reference to a standard, such as the WHO standard, and/or a percentile rank. According to the WHO 2010 standards, the threshold value for a normal semen sample is 1.5 ml.

The semen sample analysis may comprise other information related to the sample. For example, and without limitation, the analysis may comprise information on sperm DNA integrity, sex of the sperm, color, viscosity, liquefaction, pH, agglutination, presence of neutrophils, temperature, time since last previous emission, and method of production (e.g., masturbation, coitus interruptus, etc.) The analysis of such information may comprise reference to a standard value.

An established metric for sperm quality is sperm DNA integrity, i.e. a measure of sperm chromatin organization/architecture, chromatin compaction and DNA strand integrity. High DNA integrity is linked to both improved fertilization rates and embryo development in IVF and ICSI. Both density gradient centrifugation and swim-up have been found to generally select sperm populations with increased DNA integrity (as compared to the raw sample). However, density gradient centrifugation has also been shown to potentially induce sperm DNA.

Alternatively or additionally, some embodiments may not use chemicals nor include steps that might be harmful to sperm (e.g. centrifugation which can cause DNA damage).

The design of the LC-PolScope for birefringence imaging is similar to that of the traditional polarized light microscope except for two major modifications:
First, the specimen is illuminated with near circular polarized light, while in a traditional polarized light microscope, the specimen is illuminated with linearly polarized light, in which case the images display only those anisotropic structures that have a limited range of orientations with respect to the polarization axes of the microscope. In the LC-PolScope, however, a circular analyzer and a controllable circular/elliptical polarizer, enable the instrument to detect all the fibers in all directions with very high sensitivity, even without any staining.
Second, the traditional compensator is replaced by a liquid crystal based universal compensator. In the LC-PolScope, the traditional crystal compensator is replaced with a set of two liquid crystal variable retarders. The two liquid crystal devices are controlled electronically by a computer and can rapidly produce any polarization state. The schematic diagram of the birefringence LC-PolScope is illustrated in the FIG (polarized optics)

Table 1 summarizes the results for DNA Fragmentation Index (DFI) and High DNA Stainability (HDS) for human and bull sperm samples. The mean, standard deviation, median, and range for 39 ROIs from 10 human samples and 135 ROIs from 24 bull semen samples are listed.

| **Table 1. DFI and HDS summary results for human and bull sperm** | | | | |
|---|---|---|---|---|
| Sperm sample | Measurement | Method | Mean (SD) | Median (Range) |
| Human | DFI | SCSA | 83.4 (39.2) | 86.9 (1.2-171.4) |
| (39 ROIs from 10 Patients) | | PolScope | 85.7 (55.7) | 87.5 (1.4-178.5) |
| | HDS | SCSA | 0.41 (0.16) | 0.45 (0.05-0.66) |
| | | PolScope | 0.42 (0.17) | 0.42 (0.1-0.82) |
| Bull | DFI | SCSA | 98.2 (46.3) | 109 (0.2-179.8)) |
| (135 ROIs from 24 samples) | | PolScope | 98.6 (53) | 109 (3.5-178) |
| | HDS | SCSA | 0.49 (0.19) | 0.5 (0.09-0.85) |
| | | PolScope | 0.54 (0.21) | 0.56 (0.01-0.89) |

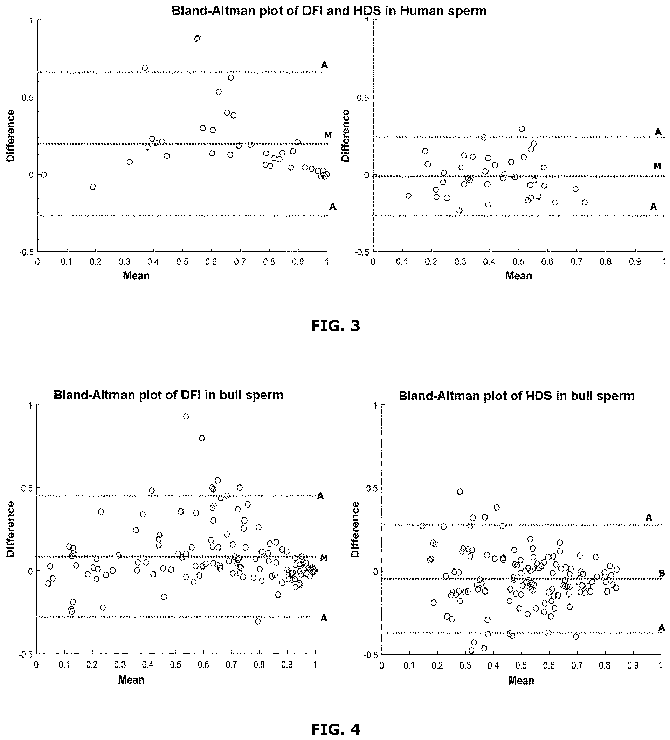

Figs. 3 depicts Bland-Altman plots of DFI and HDS for human sperm ROIs. Bland-Altman plot shows the difference between two measurements of the same parameter vs. the average of these values. "M" dotted lines show the mean and the "A" lines show the (mean ±1.96 STD), which is called the 95% confidence interval (CI).

Fig. 3 Bland-Altman plot for DFI (left) and HDS (right) in human sperm. "M" dotted lines show the mean values 0.1959 and -0.0141 for the DFI and HDS. "A" lines show the 95% confidence interval.

Fig. 4 Bland-Altman plot for DFI (left) and HDS (right) in bull sperm. "M" dotted lines show the mean values 0.0858 and -0.0458 for the DFI and HDS. "A" lines show the 95% confidence interval.

In example depicted in Fig. 3, 10 human sperm samples were used for analyses with Sperm Chromatin Assay (SCSA) and PolScope. DFI and HDS in human and bull sperm samples were measured in 2-4 ROI for each sample. The estimated correlation for DFI as measured by SCSA and PolScope in human sperm was 0.68, and the estimated correlation was 0.69 for HDS. The agreement between SCSA and PolScope for DFI and HDS in human sperm samples is good.

In example depicted in Fig. 4, 24 bull sperm samples that were evaluated using SCSA and PolScope. The estimated correlation for DFI as measured by SCSA and PolScope in bull sperm was 0.80, and the estimated correlation for HDS was 0.65. The agreement between SCSA and PolScope for DFI in bull sperm is excellent, and the agreement for HDS is good.

To obtain a birefringence image that represents the retardance and slow axis orientation in every image point, the system takes five images at five different polarization states. The first polarization setting produces circularly polarized light, and the other four settings produce elliptically polarized light with different long axis orientations. The 5 raw images are then used to generate high resolution maps of specimen retardance and slow axis orientation using image arithmetic, for instance, via implementing a logistic regression.

While in the above, a preferred embodiment has been described with reference to the accompanying drawings, the skilled person will understand that this embodiment was provided for illustrative purpose only and should by no means be construed to limit the scope of the present invention, which is defined by the claims.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

## Claims

1. A system for processing a seminal sample, the system comprising
a detecting component configured to detect sperm cells in the seminal sample,
an analyzing component configured to analyze sperm cells in the seminal sample, and
a sorting component configured to sort sperm cells in the seminal sample,
wherein the system is configured to detect health status of the sperm cells.

2. The system according to the preceding claim, wherein the system further comprises at least one of
at least one optical device comprising
at least one lens system comprising a magnifying lens, and
a proximal section and a distal section,
wherein the at least one lens system is arranged in a portion of the proximal section of the at least one optical device,
a linear polarizer,
at least two variable retarders,
a light source,
a base component comprising an insertion port configured to allow inserting the disposable microfluidic device into the base component and at least one a camera, and
a microfluidic device configured to receive at least one portion of the seminal sample.

3. The system according to any of the preceding claims, wherein the system is configured to receive a disposable cartridge comprising
a sample carrier, wherein the sample carrier comprises
a specimen holding area comprising a top side and a bottom side, wherein the top side is opposite to the bottom side,
a detachable cover configured to cover the specimen holding area, wherein detachable cover is arranged on the top side of the specimen holding area,
at least one identification mechanism comprising a barcode, and
a microfluidic element fluidly connected with the disposable cartridge,
wherein the system comprises a fluid connection between the microfluidic device and the disposable cartridge.

4. The system according to the preceding claim, wherein the system comprises at least one valve arranged between the microfluidic device and the chamber, wherein the at least one valve is configured to selectively assume at least one of at least two operational state, wherein the at least two operational states comprise a first operational state and a second, wherein the system is configured to establish a fluid connection between the microfluidic element and the disposable cartridge in the first operational state, and to discontinue the fluidic connection between the microfluidic element and the disposable cartridge while in the second operational state.

5. The system according to any of the preceding claims, wherein the microfluidic device comprises a detection zone configured to receive the at least one portion of the seminal sample and wherein the system is configured to analyzed the at least one portion of the seminal sample while the at least one portion of the seminal sample is contained in the detection zone, wherein the at least one optical device comprises at least one sensor component arranged on specimen holding at a side of the at least one lens system, wherein the at least one sensor component is configured to measure at least one signal passing through the seminal sample, wherein the at least one signal comprises a light signal, wherein the light signal passes from the seminal sample through the at least one lens system.

6. The system according to any of the preceding claims and with features of claim 2, wherein the at least one camera is configured to capture at least one image data of the specimen holding area, wherein the at least one image data comprises at least one of
a single frame image data, and
a sequence of consecutive individual frames comprising an image capture of at least 10 s⁻¹, preferably at least 90 s⁻¹, more preferably at least 300 s⁻¹, such as at least 600 s⁻¹, wherein the system comprises a first camera module configured to capture at least one first image data, and a second camera module configured to capture at least one second image, wherein the first camera module is arranged at a first position and the second camera module is arranged at a second position, wherein the first position is different from the second position, wherein the first camera module is configured to capture the at least first image data at the first position and the second camera module is configured to capture the at least second image data at the second position, wherein the first position comprises at least a portion of the first holding area and the second position comprises at least a portion of the second holding area.

7. The system according to the preceding claim, wherein the system comprises
a main circuit board comprising at least one processor unit configured to
perform at least one analytical processing on the at least one image data to generate at least one analytical finding,
determine a finding with regard to the biological specimen, wherein the finding is based on at least one the at least one analytical finding,
at least one transmitter configured to transmit data from the at least one processor unit to at least one remote device comprising at least one server, wherein the at least one transmitter is configured to transmit data comprising information of the seminal sample about at least one of
sperm count,
sperm morphology,
sperm motility, and
sperm DNA integrity,
wherein the system is further configured to
select sperm cells based on the sperm cells health status,
determine sperm concentration, sperm motility, sperm DNA integrity,
execute a morphological analysis based on the captured image data.

8. A method for analyzing a seminal sample, the method comprising
segregating sperm cells of the seminal sample using a device,
detecting the sperm cells in the seminal sample by means of a detecting component,
analyzing the sperm cells in the seminal sample by means of an analyzing component,
sorting the sperm cells in the seminal sample by means of a sorting component, and recovering the sperm cells from the device.

9. The method according to the preceding claim, wherein the method comprises
supplying a flow of the fluidic medium in a medium flow direction, wherein the medium flow direction is also defined by an initial flow point and a terminal flow point,
introducing the seminal sample into the device at the terminal flow point of the medium flow direction, recovering sperm cell from the fluidic medium at the initial flow point of the medium flow direction, and
sorting the sperm cells based on the sorting threshold.

10. The method according to the preceding claim, wherein analyzing the sperm cells comprises
capturing a first sperm cell image data of the sperm cells, wherein the first sperm cell image data comprises a first region of the sperm cell,
capturing a second sperm cell image date of the sperm cells, wherein the second sperm cell image data comprises a second region of the sperm cell, and
capturing a n-th sperm cell image data of the sperm cells, wherein the n-th sperm cell image data comprises a n-th region of the sperm cell,
wherein
analyzing the sperm cells further comprises contrasting each of the sperm image data against a corresponding reference image data,
capturing the cell image data is executing automatically using a capturing component, and
contrasting each sperm image data against a corresponding reference image data comprises using a computer-implemented analytical module, wherein the analytical computer-implemented analytical module automatically analyzes the image data via different deep learning models.

11. The method according to any of the preceding method claims, wherein the method further comprises automatically
generating a report data set comprising at least one of: sperm concentration, sperm morphology, total motility (MSC: Motile Sperm Concentration), progressive Motility (PMSC: progressive Motile Sperm Concentration), and sperm DNA fragmentation,
determining the sperm cell quality comprises determining at least one of: a number of sperm cells in the ejaculate, a percentage of sperm cells with normal morphology, a percentage of motile sperm cells, a percentage of sperm cells with low DNA fragmentation, and a numeric and a visual presentation of the results including at least one representative image data,
generating at least one of: a sperm cell quality profile, and a sperm cell quality report,
assessing sperm DNA damage,
analyzing a birefringence value comprising sperm nuclear integrity levels,
executing a reference analytical procedure, wherein the reference analytical procedure comprises at least one quality control parameter comprising at least one of
sperm dynamic data,
sperm DNA integrity,
concentration, and
morphology

12. The method according to any of the preceding method claims, wherein the method comprises
receiving at least one disposable cartridge in the system,
receiving at least one portion of the seminal sample in the device,
implementing at least one identification mechanism via the at least one identification mechanism, and
receiving at least one portion of the seminal sample in a detection zone of the device, and analyzing the at least one portion of the seminal sample while the at least one portion of the seminal sample is contained in the detection zone.

13. The method according to any of the preceding method claims, wherein the method comprises
measuring at least one signal passing through the seminal sample via the at least one sensor component, wherein the at least one signal comprises a light signal,
capturing at least one first image data by means of a first camera module, and at least one second image data by means of a second camera module,
performing at least one analytical processing on the at least one image data to generate at least one analytical finding,
determining a finding with regard to the biological specimen, wherein the finding is based on at least one the at least one analytical finding,
transmitting a light signal from at least one light source through the specimen holding area, and
transmitting data from the at least one processor unit to at least one remote device by means of at least one transmitter comprising information of the seminal sample about at least one of: sperm count, sperm morphology, sperm motility, and sperm DNA integrity.

14. The method according to any of the preceding method claims, wherein the method comprises
determining at least one of sperm concentration, sperm morphology, sperm motility, sperm DNA integrity,
automatically executing auto-calculation by means of an artificial intelligence-based module,
capturing image data with a capture-rate of at least 100 frames per second, at least 660 frames per second, at least 200 frames per second,
executing a morphological analysis based on the captured image data,
indicating a percentile rank for at least one of: sperm number, sperm morphology, sperm motility, sperm DNA integrity and composite value for the seminal sample, and
indicating whether the seminal sample is considered fertile or sub-fertile.

15. The method according to the 2 preceding claims, wherein the method comprises determining at least one threshold value for at least one of: total motility percentage, progressive motility percentage, progressive velocity, motility, and viability, and
the sperm motility comprises an analysis of at least one of: total motility percentage, progressive motility percentage, motile sperm per ejaculate, progressive velocity, mean linearity, motility, viability percentage, and classification of motility as rapid, medium, or slow,
the sperm morphology comprises an analyzing at least one of: analysis of head size such small, normal, or large, head shape such as round, tapered, amorphous, midpiece shape and size, tail shape and size, duplicate forms such as multiple heads, multiple tails, and cytoplasmic droplets, and
the seminal sample analysis comprises at least one of: sperm DNA integrity, sex of the sperm, color, viscosity, liquefaction, pH, agglutination, presence of neutrophils, temperature, time since last previous emission, and method of production such as masturbation, coitus interruptus.
